# EUROPEAN PATENT APPLICATION

(11) **EP 1 714 673 A1**
(43) Date of publication of application: **25.10.2006**
(21) Application number: 05075948.9
(22) Date of filing: 21.04.2005
(51) Int. Cl.: A61P 3/02, A23K 1/18, A23K 1/165, A23K 1/16

(54) **Method for lengthening the mobility period of fish**

(71) Applicant: Desol BV, 6167 RA Geleen (NL)
(72) Inventor: Elshout van den, Wilhelmus Hubertus Henricus A., 6167 RA Geleen (NL); Forier, Rudi Ludovicus Florent, 3520 Zonhoven (BE)
(74) Representative: Habets, Winand

(57) **Abstract**

This invention is in the field of animal feed, more in particular in the field of fish feed. Surprisingly, it has been found that the mobility period of fish may be lengthened by interfering with the diet of the fish. We found that it is possible to increase the mobility period of fish to such an extent that it is even active at temperatures as low as 2 degrees Celsius. Such may be accomplished by adding naturally occurring substances to the feed of the fish. Examples of these naturally occurring substances are immune enhancing ingredients like beta-glucans and/or small amounts of phytohormones like auxin or gibberellic acid. Also, combinations of these substances, in particular the combination of beta-glucans and phytohormones, were shown to have a synergistic effect in that they lengthened the mobility period of the fish more than the individual components on their own.

## Description

This invention is in the field of animal feed, more in particular in the field of fish feed.

Fish feed comes in a large variety of substances. It is essential that such feed contains all the necessary nutrients for the animal in order to grow well and protect the animal from disease.

It is a well known phenomenon that many fish species have a period of rest when the temperature of the water or the length of the day drops below a certain value. During that period of reduced mobility, the metabolism of the fish decreases dramatically. As a consequence, the fish will not consume food and will stay in the immobile phase as long as the temperature and/or daylight conditions are maintained at the sub-critical level. When the temperature of the water and/or the length of the day increases again, for instance due to seasonal changes, the fish will become mobile and will start eating again.

This mechanism has been extensively studied for koi fish for instance. Koi will become immobile when the temperature of the water drops below about 8 degrees Celsius. The mobility of the fish greatly reduces and it will descend to deeper waters and stay there as long as this condition is maintained. When in the spring the water temperature rises above 8 degrees Celsius, the fish will become mobile again and will occasionally start to swim to the surface.

Koi fish owners have tried to increase the period of activity or mobility of their koi for obvious reasons. It is a well known phenomenon that the temperature of the air rises more quickly in the spring than the temperature of the water. Hence, the koi owners would want their fish to surface at approximately the same time when they themselves feel like recreating outside.

One of the obvious solutions provided for this problem is to heat the water by using artificial heating devices so that the temperature of the water does not drop below the critical value. It has also been suggested to artificially lengthen the day so that koi may stay mobile for a longer period of time. It goes without saying that such may be costly and energy-consuming solutions especially with large outdoor pools in cold climates.

Surprisingly, it has now been found that the mobility period of fish may be lengthened by interfering with the diet of the fish. We found that it is possible to increase the mobility period of fish to such an extent that it is even active at temperatures as low as 2 degrees Celsius. Such may be accomplished by adding naturally occurring substances to the feed of the fish.

Examples of these naturally occurring substances are immune enhancing ingredients like beta-glucans and/or small amounts of phytohormones like auxin or gibberellic acid. Also, combinations of these substances, in particular the combination of beta-glucans and phytohormones, were shown to have a synergistic effect in that they lengthened the mobility period of the fish more than the individual components on their own.

Phytohormones are herein defined as molecules that function to coordinate plant growth and development. The compounds that have been considered as plant hormones are for instance: indole-3-acetic acid (auxin), cytokinin, gibberellin, ethylene, abscisic acid. In addition, brassinosteroids, jasmonic acid and salicylic acid have been shown to have important growth regulating activities and are considered to function as Phytohormones.

Particularly good results were obtained when the fish feed was supplemented with free IAA instead of conjugated IAA. Free IAA and conjugated IAA are known compounds. free IAA is a naturally-occurring plant growth phytohormone which has been extensively studied. In plants, most of the IAA occurs in a conjugated form (Slovin et al. 1999, Biochemistry and molecular biology of plant hormones, Elsevier, Amsterdam. P115-140), either conjugated to sugars via ester linkages or to amino acids and peptides via amide linkages.

Free IAA is readily available as a commercial product. It may be synthesised chemically or prepared in a biological way. IAA producing micro-organisms are widespread in nature. Yeast, fungi and many bacteria as well as plants are known to convert precursors of IAA into free IAA. In addition to the L-tryptophan conversion by bacteria, also L-tryptophan independent biochemical routes towards free IAA are described extensively (J. Plant Growth Regul (2001) 20: 198-216).

A well known bacterium, capable of producing free IAA is Azospirillum Brasilense (AB). At the end of the growth phase in a regular fermentation process, AB is able to convert L-tryptophan into free IAA. To increase the efficiency of this conversion, a small amount of synthetic free IAA may be added to the media. Via a feedback mechanism, AB increases the conversion of L-tryptophan into free IAA.

Final concentrations of 1 gram free IAA / liter culture broth are easy to make, but even much higher concentrations are possible, depending on the micro-organism used.

After ending the fermentation the micro-organism may be lysed and a powder enriched in free IAA may be obtained by spray drying or any other convenient way of drying the culture broth. Other techniques may be used to remove liquids partly or completely.

The term "free IAA" is used herein to indicate that the free IAA is in the free or acid form, whereas the term "conjugated IAA" refers to IAA that is conjugated via ester linkages or via amide linkages.

As long ago as 1956, the effects of free IAA on humans were studied, and it was shown that single doses of 0.1 g/kg were non-toxic (Mirsky A and Diengott D, Hypoglycemic action of indole-3-acetic acid by mouth in patients with diabetes mellitus, Proc. Soc. Exp. Biol. Med. 93: 109-110,1956)- In 1964, it was found that photo-oxidation products of free IAA acted as growth inhibitors of micro-organisms (Still C, Fukuyama T and Moyed H, Inhibitory Oxidation Products of Indole-3-acetic acid, J. Biological Chemistry, 240,6,2612-2618, 1964).

Also, the medical use of free IAA and some of its derivatives has previously been described. EP 1.296.676 describes the use of free IAA as a pharmaceutical, in particular for treating neoplastic disease in humans. WO 02/080906 describes the use of free IAA for treating endometriosis in women. Nachson et al. (Food and Chemical Toxocology 41, 745-752) reported the effect of some free IAA derivates (indole-3-carbinol and 3,3'-diindolylmethane) on the proliferation and induction of apoptosis in human prostate cancer cell lines whereas Rossiter et al. (Bioorganic & Medicinal Chemistry Letters, 12, 2523-2526) as well as Folkes et al. (Biochemical Pharmacology 63, 265-272) described the use of free IAA and some derivatives in enzyme-prodrug directed cancer therapies.

It has now been found that Phytohormones and beta-glucans work in a wide range of concentrations for lengthening the mobility period of fish. The optimal concentrations may vary somewhat between different species of fish, however, the skilled person will know how to obtain an optimal concentration for a given species, for instance by titration of the desired compound into the fish feed and testing when this would have the optimal effect. The following may serve as guidance in this process.

A skilled person will appreciate that the amount of free IAA in the ready to use feed has to be adjusted in order to supply the animal with an effective amount of free IAA. In order to adjust the free IAA concentration in the feed so that a certain daily intake of free IAA is achieved, an estimate has to be made of the feed intake of an animal or animal group. A skilled person is aware of the feed intake of a (particular kind or group of) animal(s), usually the feed intake per day is between 0,05 and 10% of the body weight of the animal, with occasional exceptions as high as 20%. Koi awakening from their resting period, however, consume much less, in the order of 0,01 to 0,1% of their body weight.

Koi fish responded with increased mobility when free IAA was provided in the range of 0,02 and 240 microgram per kilogram life weight per day (ug/kglw/day). Optimum between cost and benefit was reached in concentrations between 0,2 and 24 ug/kglw/day, in particular feed containing between 1 and 5 ug/kglw/day such as 2,4 ug/kglw/day free IAA was very effective.

It was also found that within the family of beta-glucans, in particular 1,3 and 1,6 beta glucans were very useful to lengthen the mobility period of fish. A particularly good source of such 1,3 and 1,6 beta glucans may be found in preparations of Agaricus blazei murill (ABM) or in yeast cell walls. Fresh ABM or dried ABM may be used in the invention, dried ABM is usually 10% of the weight of fresh ABM. Dried ABM may contain in the order of 2 to 50% of 1,3 and/or 1,6 beta glucans, typically in the order of 10 to 20%.

Fish food supplemented with 1 to 10,000 ug/kglw/day of dried Agaricus blazei murill (corresponding to 10 to 100,000 ug fresh Agaricus blazei murill per kglw/day) was found to produce the desired effect of lengthening the mobility period of fish. This corresponds to approximately 0,1 to 2000 ug/kglw/day of pure 1,3 and 1,6 beta glucans. Excellent results were obtained with fish feed containing 10 to 200 ug/kglw/day of pure 1,3 and 1,6 beta glucans, optimum of cost benefit was found to be around 60 ug/kglw/day of pure 1,3 and 1,6 beta glucans or 600 ug/kglw/day of dried Agaricus blazei murill.

Koi fish also showed the desired effect when their food was supplemented with Gibberelin or Gibberellic acid. The optimal concentrations here were found to be within the range of 0,002 and 20 ug/kglw/day. In the range of 0,02 and 2 ug/kglw/day was the effect of lengthening the mobility period of fish particularly pronounced. Optimal results were achieved between 0,1 and 2 ug/kglw/day, such as 0,24 ug/kglw/day.

### Examples

### Example 1: Microbiological production of a preparation containing free IAA

Azospirillum brasilence Sp7 (ATCC) was obtained as an agar culture in a culture tube. LB medium was used to grow the strain overnight at 28 °C at 175 rpm. Glycerol was added to the culture up to 10 %, mixed and divided over Nalgene creovials and frozen at - 80°C. Stocks were stored at - 80 °C in creovials.

To prepare a seed culture of A. brasilence, one stock (1.2 to 1.8 ml) was thawed and added to 1 liter of LB medium and grown for about 20 h at 28 °C and 175 rpm to an Optical Density (OD620 nm) of about 2.5.

A 10 litre fermentor was rinsed with water and the pH electrode was calibrated. Nine litre of LB medium was prepared and 1 g/l L-Tryptophan and 0.1 g/l free IAA was added. The medium was entered into the fermentor together with 2 ml of anti foam. The fermentor was sterilised for 30 min at 121 °C. After cooling down to 28 °C, the 02 probe is calibrated with N2 and 02, 0 and 100 % air saturation respectively_

The seed culture is transferred to the fermentor via a flask and tubing which are separately sterilised in an autoclave. When the addition is completed the tubing and flask are removed and the fermentation is started with the following parameters:

| | |
|---|---|
| Stirrer speed | 400 rpm |
| Temperature | 28 °C |
| Aeration | 0.75 Nl/min |
| PH | 7 |

After 15 min a sample is taken to measure the OD620 nm and check the pH. Samples are taken at certain intervals to quantify the growth of A. brasilence. When the growth rate declined extra medium was added to ensure that enough biomass was formed for the production of free IAA. It was found that the production of free IAA started when the active growth phase ended and continued for a prolonged period. The course of the free IAA concentration was followed by LC-MS. When the concentration of free IAA was at a level of about 1 g/l, the fermentation was terminated and the cells were harvested and lysed by means of a nonojet homogeniser at about 1400 bar. The remaining supernatant and the lysed cells were sterilised and spray dried to yield the desired product formulation.

### Example 2: Preparation of feed containing beta glucans

An amount of 3,0 gram of Agaricus Blazei Murill (Agaricus Farm), a natural source of beta-glucans was suspended in 100 ml of olive oil. A fish feed according to the invention was prepared by vacuum impregnating one kilogram of commercially available (Coppens) Cyprico White 3 mm floater feed with 100 ml of the oil suspension. Control feed was prepared by vacuum impregnating the same amount of feed with only olive oil.

### Example 3: Preparation of feed containing plant growth hormones

An amount of the spray dried formulation as described in example 1 corresponding to 12 milligram of free IAA was suspended in 100 ml of olive oil. A fish feed according to the invention was prepared by vacuum impregnating one kilogram of commercially available (Coppens) Cyprico White 3 mm floater feed with 100 ml of the oil suspension. Control feed was prepared by vacuum impregnating the same amount of feed with only olive oil.

### Example 4: Preparation of feed containing both beta glucans and plant growth hormones

An amount of 3,0 gram of Agaricus Blazei Murill (ABM, Agaricus Farm), a natural source of beta-glucans and an amount of the spray dried formulation as described in example 1 corresponding to 12 milligram of free IAA were suspended in 100 ml of olive oil. A fish feed according to the invention was prepared by vacuum impregnating one kilogram of commercially available (Coppens) Cyprico White 3 mm floater feed with 100 ml of the oil suspension. Control feed was prepared by vacuum impregnating the same amount of feed with only olive oil.

### Example 5: Use of fish feed comprising beta glucans and plant growth hormones to lengthen the period of mobility of fish

Four ponds of 40 cubic meters each, all contained 50 koi fish with an approximate total body weight of 50 kg were used to show effectiveness of the fish feed according to the invention. One pond served as a control were the fish were fed with control feed, the fish in the other ponds received a feed according to the invention as prepared in examples 2 to 4 above. The experiment started when the temperature of the water was about 2 degrees Celsius.

The fish in control pond were fed with 10 gram Cyprico White 3 mm floater feed per day, whereas the fish in the other pond were fed with 10 gram of the feeds as described in Example 2, 3 and 4 Table 1).

It was observed that the fish in the test groups swam to the surface and started to eat the feed whereas the control group showed no sign of activity. This effect increased gradually every day until after 5 days the fish were fully mobile, whereas the temperature of the water was still between 2 and 3 degrees Celsius. Mobility of the fish was scored on a relative index of 1 to 5 wherein 1 is fully immobile and 5 is fully mobile Table 2). The water in the control pond was at exactly the same temperature as that of the other ponds, however the fish in the control pond were completely immobile. After four weeks, when the water temperature rose gradually to 6 degrees Celsius, no adverse side effects were observed in the test group. The fish were healthy and fully mobile whereas the control group was almost fully immobile.

**Table 1**

| | Feed additive |
|---|---|
| Pond 1 | 2,4 ug/kglw/day free IAA |
| Pond 2 | 600 ug/kglw/day ABM |
| Pond 3 | 2,4 ug/kglw/day free IAA plus 600 ug/kglw/day ABM |
| Pond 4 (control) | none |

**Table 2**

| Day | Temperature [degrees Celsius] | Mobility in Pond 1 | Mobility in Pond 2 | Mobility in Pond 3 | Mobility in Pond 4 |
|---|---|---|---|---|---|
| **1** | **2** | **1** | **1** | **1** | **1** |
| **2** | **2** | **1** | **1** | **2** | **1** |
| **3** | **3** | **1** | **1** | **2** | **1** |
| **4** | **3** | **2** | **2** | **2** | **1** |
| **5** | **3** | **2** | **2** | **3** | **1** |
| **8** | **3** | **3** | **1** | **2** | **1** |
| **12** | **3** | **2** | **2** | **3** | **1** |
| **15** | **4** | **2** | **2** | **4** | **1** |
| **20** | **5** | **3** | **3** | **4** | **1** |
| **24** | **5** | **4** | **4** | **5** | **2** |
| **28** | **6** | **4** | **4** | **5** | **2** |

| | | | | | |
|---|---|---|---|---|---|
| Table 2 shows the relation between mobility of fish expressed on a scale of 1 to 5 (1 is fully immobile, 5 is fully mobile) and the temperature of the water in the pond. Fish in ponds 1, 2 and 3 were fed with fish feed according to the invention, fish in pond 4 received control feed. Further details are described in the examples. | | | | | |

## Claims

1. Fish feed comprising between 0,015 and 150 gram of beta-glucans per kilogram of feed.

2. Fish feed according to claim 1 comprising between 0,015 and 15 grams, preferably between 0,1 and 1 gram of beta-glucans per kilogram of feed.

3. Fish feed according to claims 1 or 2 wherein the beta glucans comprise 1,3 and/or 1,6 beta glucans.

4. Fish feed comprising between 0,3 and 3000 milligram of phytohormones per kilogram of feed.

5. Fish feed according to claim 4 comprising between 3 and 300 milligrams, preferably between 5 and 100 grams of phytohormones per kilogram of feed.

6. Fish feed according to claims 4 or 5 wherein the phytohormone is free IAA.

7. Fish feed according to claims 4 or 5 wherein the phytohormone is gibberellic acid.

8. Fish feed according to claims 1 and 4.

9. Use of a fish feed according to claims 1 to 8 for lengthening the mobility period of fish.

10. Method of impregnating a fish feed with a phytohormone and/or a beta glucan using vacuum extraction.
